# EUROPEAN PATENT SPECIFICATION

(11) **EP 3 709 895 B1**
(45) Date of publication and mention of the grant of the patent: **01.01.2025**
(21) Application number: 18830539.5
(22) Date of filing: 12.11.2018
(51) Int. Cl.: A61B 10/02

(54) **A DEVICE TO PERFORM THE BIOPSY OF SOFT (USUALLY HUMAN) TISSUE**
VORRICHTUNG ZUR DURCHFÜHRUNG EINER BIOPSIE VON WEICHEM (MEIST MENSCHLICHEM) GEWEBE
DISPOSITIF DE MISE EN OEUVRE DE LA BIOPSIE D'UN TISSU MOU (GÉNÉRALEMENT HUMAIN)

(30) Priority: 13.11.2017 PL 42344017
(43) Date of publication of application: 23.09.2020
(73) Proprietor: Gdanski Uniwersytet Medyczny, 80-210 Gdansk (PL); Politechnika Gdanska, 80-233 Gdansk (PL)
(72) Inventor: ZIELINSKI, Jacek, 83-110 Tczew (PL); JÓZEFIAK, Lech, 80-288 Gdansk (PL); WTOREK, Jerzy, 80-180 Jankowo (PL); BUJNOWSKI, Adam, 80-180 Jankowo (PL)
(74) Representative: JWP Patent & Trademark Attorneys
(86) International application number: PCT/PL2018/000110
(87) International publication number: WO 2019/093914

(56) References cited:
- US-A- 5 197 484
- US-A- 5 795 308
- US-A1- 2001 014 778
- US-A1- 2003 045 873

## Description

### Technical field

The subject of the invention relates to a device to perform the biopsy of soft, especially human tissue, wherein the tissue is further subject of microscopic examinations.

### Prior art

There is a known device to perform a fine needle biopsy - Fine Needle Aspiration Biopsy FNAB - with the use of a fine needle and a syringe drawing in the content of a tumor e.g. of a thyroid or a lymph node, what allows only to take a sample of single cells.

There are also known devices to perform a biopsy of e.g. cancerous tumors which are equipped in an energy storage and a suitably compressed set of coaxially connected needles. After selecting a place to take a sample of tissue, the energy storage is primed what results in drawing the external needle. In this position, both suitably shaped needles form a cutting system and an open chamber to which the tissue is inserted under the systemic pressure. After releasing the energy from the energy storage, the external needle rapidly returns to the starting position inserting the tissue into the external needle. Depending on the producer these devices are either disposable or reusable.

From US 5795308 it is known a device and method for coaxial breast biopsy utilizing a rigid rod. The device comprising a rigid rod which has retractable anchor hooks and a spiked collar to slide over the rod. The cancer is bracketed by the anchor hooks distally and the spiked collar proximally to stabilize it on the rod and mark its position for excision by the cannula.

Patent application US2003/045873 A1 discloses a surgical apparatus for removing a portion of tissue comprising an elongated body having a distal edge, a collapsible cutting member mounted to the elongated body, having an opening therethrough and extending distally of the distal edge of the elongated body. The cutting member resects a tubular region of tissue as the apparatus is advanced through tissue and the resected tissue extends through an opening in the cutting ring for containment within the elongated body. The cutting member has a closed loop conductive surface which forming an electrosurgical cutting surface for applying electrical energy to tissue.

Patent application US2001/014778 A1 discloses a biopsy device which includes a localization needle having a guide wire preloaded into the biopsy device, a stylet having a blade for transecting and separating tissue and a cannula for cutting a core of tissue and a garrote wire mechanism for cutting a transection of tissue transverse to the core cut by the cannula.

Patent application US5197484 A discloses an apparatus for removing suspect breast tissue which includes a penetrating member in the form of a stylet with a tapered front end that can be guided along a localization guide wire. The apparatus also including a first cutting device in the form of a cannula slidingly engaged with the stylet.

One of the inconveniences of these devices is the small dimension of the collected tissues which leads to the necessity to undertake between 5 and 10 pricks in order to obtain the appropriate image from the border of a pathological lesion and a healthy tissue. This, in turn, results in the prolongation of the time of the procedure and the deepening the discomfort of an examined patient.

There is also a device for a mammotome biopsy - Vaccum Assistant Biopsy VAB, which uses the ultrasound device with a cutting part which collects the tissue samples in the form of a cell suspension, and not essential fragments of a healthy and pathological tissue.

### Summary of the Invention

The aim of the invention is to develop a device to collect a possibly optimal sample from a soft, especially human tissue, including the tissues from the border between healthy and pathological tissues.

According to the invention, a device to perform a biopsy of soft, especially human tissue comprising a cutting system consisting of a cutting sleeve with a cutting edge located on a cutting bit which can be detached, on the internal surface of a cutting sleeve, close to the cutting bit, there is a circumferential channel which is connected by a pass-through hole with a longitudinal guiding groove which is formed on the external surface of the cutting sleeve, wherein in the circumferential channel, in a pass-through hole and in in the longitudinal guiding groove there is a flaccid tie whose ends led outside of the cutting system, characterized in that there is an expanding is inserted to in the inside of the cutting sleeve, wherein the expanding sleeve enables the formation of a cutting loop from the middle part of the flaccid tie.

In the device according to invention the cutting edge has cutters.

The device according to the invention is characterized by a drive system (N) to which the end of ties are led which then are led out of the cutting system.

In one embodiment according to invention the drive system consists of a pass-through hole of casing in which detachable cutting system is fitted, the casing is equipped with a bracket as well as a propeller and a locking screw, wherein the ends of the flaccid tie are led through the pass-through hole of the casing and are fixed to pins of the propeller.

In the device according to invention a longitudinal groove is in the cutting sleeve and in the casing there is a threaded pass-through hole whose axis is perpendicular to the axis of a hole of the casing, wherein in the threaded pass-through hole there are: a set screw and a locking screw which are fitted through and opposite.

In the device according to invention there is a protective sleeve fitted on the cutting sleeve.

In the device according to invention there is an external sleeve around the cutting sleeve forming an internal sleeve, wherein the inside of the external sleeve, the cutting bit as well as the outside of the internal sleeve form the circumferential channel.

In the device according to invention the cutting bit is detachable.

The device according to the invention facilitates performing the biopsy and ensures that during the procedure there is sufficient amount of material collected as well as optimal material from the border of a pathological lesion and a healthy tissue. The properly selected length of the cutting sleeve and a proper progressive, rotatable and oscillating depth movement allows to eliminate an open biopsy which cumbersome for the patient.

### Brief Description of the Drawings

The invention is explained in details in examples of the realization and in the drawing in which:
**Fig. 1** presents a general view of the device with the internal tie covered by the sleeve,
**Fig. 2** presents a device section A-A in a version with a unitary cutting sleeve and tie leading out to the groove of the cutting sleeve,
**Fig. 3** presents a device section B-B of the end of the cutting sleeve,
**Fig. 4** presents an axial section of the end of the cutting bit with cutters,
**Fig. 5** presents an axial section of the cutting bit with an expanding sleeve,
**Fig. 6** presents a general view of the device with an internal tie,
**Fig. 7** presents an axial section of the cutting sleeve with an internal tie and
**Fig. 8** presents an axial section of the detachable cutting bit.
**Fig. 9** presents a partial section A-A of the cutting sleeve with a groove protecting the tie.

### Examples

### Example 1

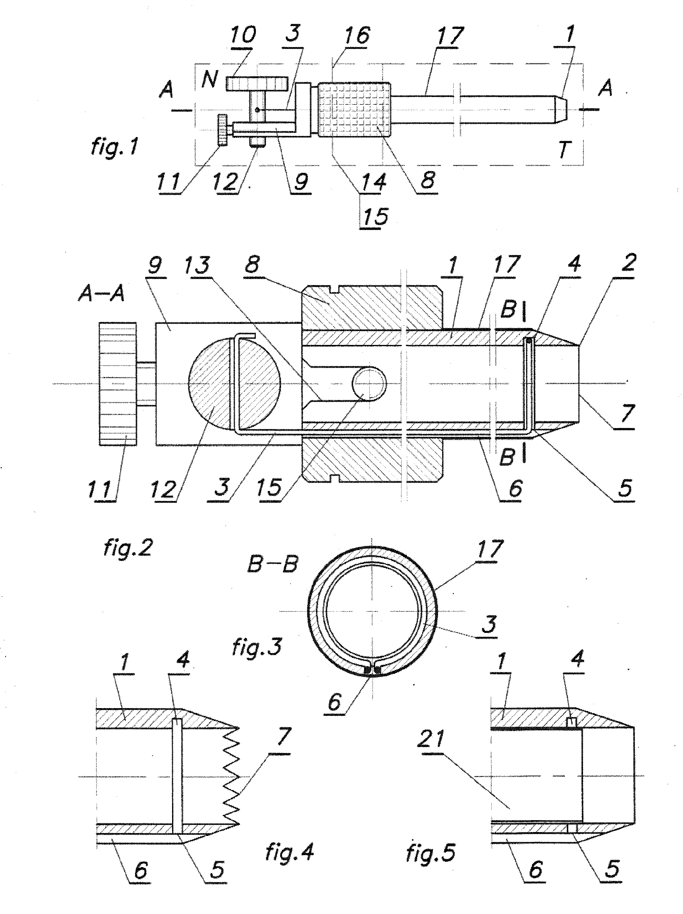

As shown in figure 1, the device consists of a cutting system T and a drive system N. As shown in figure 2 and figure 3, the cutting system T consists of a cutting sleeve 1 with a cutting edge 2. There are cutters 7 on the cutting edge. On the internal surface of the cutting sleeve 1, in the proximity of the cutting edge 2, there is a circumferential channel 4, which is connected through a pass-through hole 5 with a longitudinal groove 6 fitted on the external surface of the cutting sleeve 1. In a circumferential channel 4, in a pass-through hole 5 and in a longitudinal groove 6 there is a tie 3. The ends of the tie 3 are led through the longitudinal groove 6 outside the cutting sleeve 1. As shown in figure 1 and figure 2 a drive system N consists of a casing 8, which has a pass-through hole and is equipped with a bracket 9. The bracket 9 has a propeller 10 as well as a locking screw11.

In the casing 8 there is a cutting system T which is non-detachable.

The ends of the tie 3 are led through the longitudinal groove 6 and the pass-through hole of the casing 8 and are fixed to the pins 12 of the propeller 10.

According to the medical practice, the cutting edge is placed towards the patient's body and is inserted to the proper depth. Then, the ends of the tie 3 are pulled and the cutting loop cuts off a section of soft tissue.

### Example 2

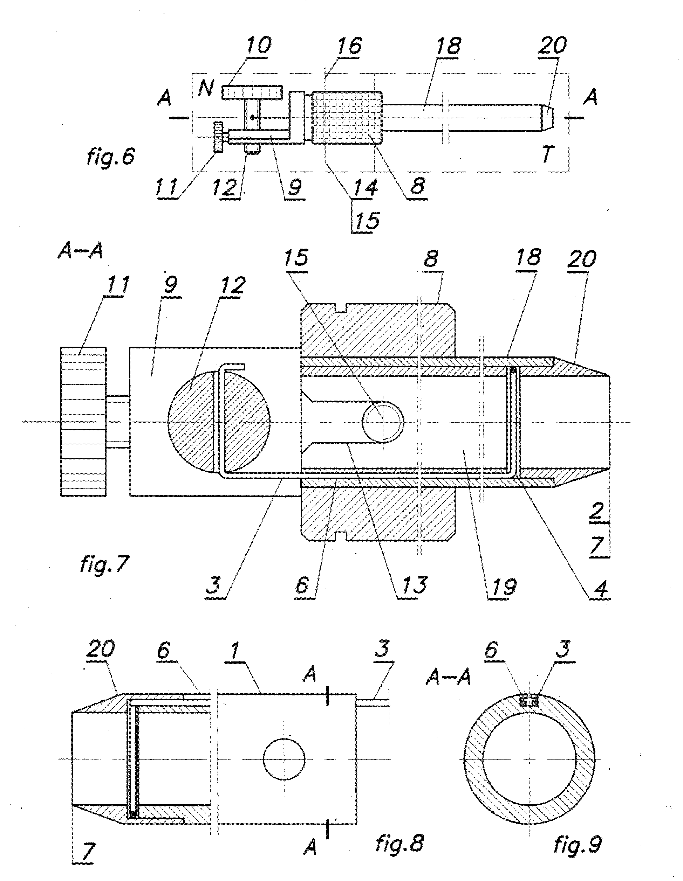

As shown in figure 7, the cutting system T consist of an external sleeve 18, an internal sleeve 19, a cutting bit 20 and a tie 3. In the external sleeve 18 there is a cutting bit 20 with a cutting edge 2. The inside of the external sleeve 18, the cutting bit 20 as well as internal sleeve 19 form a circumferential channel 4, which is connected with a longitudinal groove 6 on the internal sleeve 19.

Depending on the version, a longitudinal groove 6 is fitted on the internal sleeve 19, external sleeve 18 or on both of these sleeves. In a circumferential channel 4 and in a longitudinal groove 6 there is a tie 3. The ends of the tie 3 go outside of the cutting system T between the external sleeve 18 and internal sleeve 19.

On the cutting edge 2 there are cutters 7.

The device is equipped with a drive system N, which is constructed as shown in example 1.

In the external sleeve 18 and in the internal sleeve 19 there is a channel 13, and in the casing 9 there is a threaded pass-through hole 14, whose axis is perpendicular to the axis of a hole of the casing 8. In the threaded pass-through hole 14 there are: a set screw 15 and a locking screw 16 which are fitted through and opposite.

A set screw 15 and a locking screw 16 ensure a unique position in the casing 8.

### Example 3

The device is constructed as in example 1 but the cutting system T, as shown on figure 8, consists of a cutting sleeve 1 and a detachable cutting bit 20 located on the sleeve. The circumferential channel 4 was formed by a proper selection of measurements of the cutting sleeve 1 a cutting bit 20. The cutting bit 20 is equipped with cutters 7.

Figure 9 presents a section A-A of the cutting sleeve 1 with an exemplary shape of the groove 6 protecting the tie 3.

Before the assembly of the device, first the tie 3 is formed in a shape described in example 1. The assembly of the cutting system T consists of inserting the tie 3 into the cutting bit 20 and then connecting these elements with the cutting sleeve 1.

### Example 4

The device is constructed as in example 1 but as shown in figure 2, on the cutting sleeve 1 a protective sleeve 17 is fitted. A sleeve 21 is inserted into a cutting sleeve as shown in figure 5.

An expanding sleeve 21 enables the formation of a cutting loop from the middle part of the tie 3 made from a flaccid material.

The ends of the tie 3 are led through a pass-through hole 5 and a longitudinal groove 6 to the outside of the cutting system T.

The biopsy of the soft tissues is performed as shown on the example 1. However, before the procedure, the protective sleeve 17 is removed from the cutting sleeve 1.

After inserting the cutting system T into the patient's body to a selected depth, the expanding sleeve 21 is moved to such an extent so as to expose the cutting loop of a tie 3 and then the ends of the tie 3 are pulled, what causes the tightening of the loop and taking a sample of tissue.

## Claims

1. A device configured to perform a biopsy of soft, especially human, tissue having a cutting system (T) consisting of a cutting sleeve (1) with a cutting edge (2) located on a cutting bit (20) which can be detached, on the internal surface of the cutting sleeve (1), close to the cutting bit (20), there is a circumferential channel (4) which is connected by a pass-through hole (5) with a longitudinal guiding groove (6) which is formed on the external surface of the cutting sleeve (1), wherein in the circumferential channel (4), in the pass-through hole (5) and in the longitudinal guiding groove (6) there is a tie (3) whose ends are led outside of the cutting system (T) to the drive system (N) wherein on the cutting sleeve (1) there is a protective sleeve (17) fitted and an expanding sleeve (21) is inserted within the cutting sleeve (1), **characterized in that** the tie (3) is flaccid and the expanding sleeve (21) enables the formation of a cutting loop from the middle part of the flaccid tie (3).

2. The device according to claim 1, wherein the drive system (N) comprises a pass-through hole in a casing (8) in which detachable cutting system (T) is fitted, the casing (8) is equipped with a bracket (9) as well as a propeller (10) and a locking screw (11), wherein the ends of the flaccid tie (3) are led through the pass-through hole of the casing (8) and are fixed to pins (12) of the propeller (10).

3. The device according to claim 2, wherein a longitudinal groove (13) is in the cutting sleeve (1) and in the casing (8) there is a threaded pass-through hole (14) whose axis is perpendicular to the axis of a hole of the casing (8), wherein in the threaded pass-through hole (14) there are: a set screw (15) and a locking screw (16) which are fitted through and opposite.

## Patentansprüche

1. Vorrichtung zur Durchführung einer Biopsie von weichem, insbesondere menschlichem, Gewebe, mit einem Schneidsystem (T), das aus einer Schneidhülse (1) mit einer Schneideklinge (2) besteht, die sich auf einem abnehmbaren Schneidestück (20) befindet, auf der Innenfläche der Schneidhülse (1), in der Nähe des Schneidestücks (20), befindet sich ein Umfangskanal (4), der durch ein Durchgangsloch (5) mit einer Längsführungsnut (6) verbunden ist, die auf der Außenfläche der Schneidhülse (1) ausgebildet ist, wobei sich im Umfangskanal (4), im Durchgangsloch (5) und in der Längsführungsnut (6) ein Zugband (3) befindet, dessen Enden außerhalb des Schneidsystems (T) zum Antriebssystem (N) geführt werden, wobei auf der Schneidhülse (1) eine Schutzhülse (17) angebracht ist und eine Spreizhülse (21) in die Schneidhülse (1) eingesetzt ist, **dadurch gekennzeichnet, dass** das Zugband (3) schlaff ist und die Spreizhülse (21) die Bildung einer Schneideschleife aus dem mittleren Teil des schlaffen Zugbandes (3) ermöglicht.

2. Vorrichtung nach Anspruch 1, wobei das Antriebssystem (N) ein Durchgangsloch in einem Gehäuse (8) umfasst, in dem ein abnehmbares Schneidsystem (T) angebracht ist, das Gehäuse (8) mit einem Halter (9) sowie einer Antriebsschraube (10) und einer Sicherungsschraube (11) ausgestattet ist, wobei die Enden des schlaffen Zugbandes (3) durch das Durchgangsloch des Gehäuses (8) geführt und an Stiften (12) der Antriebsschraube (10) befestigt werden.

3. Vorrichtung nach Anspruch 2, wobei in der Schneidhülse (1) eine Längsnut (13) vorhanden ist und im Gehäuse (8) sich ein Gewindedurchgangsloch (14) befindet, dessen Achse senkrecht zur Achse eines Lochs des Gehäuses (8) verläuft, wobei in dem Gewindedurchgangsloch (14) Folgendes vorhanden ist: eine Feststellschraube (15) und eine Sicherungsschraube (16), die durchgehend und gegenüberliegend angebracht sind.

## Revendications

1. Dispositif configuré pour effectuer une biopsie de tissu mou, particulièrement de tissu humain, ayant un système coupante (T) consistant au manchon coupant (1) avec un bord coupant (2) situé sur un trépan tranchant (20) qui peut être détaché, sur une surface intérieure du manchon coupant (1), près du trépan tranchant (20), il y a un canal circonférentiel (4) qui est relié par un trou de traversée (5) avec une rainure de guidage longitudinale (6) qui est formée sur une surface extérieure du manchon coupant (1), dans lequel il y a une entretoise (3), dont extrémités sont dirigées vers l'extérieur du système coupant (T) au système d'entraînement (N), dans le canal circonférentiel (4), le trou de traversée (5) et la rainure de guidage longitudinale (6), dans lequel il y a un manchon de protection (17) monté sur le manchon coupant (1) et un manchon extensible (21) est inséré dans le manchon coupant (1), **caractérisée en ce que** l'entretoise (3) est flasque et le manchon extensible (21) permet de former une boucle coupant à partir d'une partie moyenne de l'entretoise flasque (3).

2. Dispositif selon la revendication 1, dans lequel le système d'entraînement (N) comprend un trou de traversée (5) dans un boîtier (8), dans lequel le système coupante (T) amovible est intégré, le boîtier (8) est muni d'une saillie (9) ainsi que une hélice (10) et une vis de serrure (11), dans lequel les extrémités de l'entretoise flasque (3) sont dirigées par trou de traversée du boîtier (8) et sont attachés aux goupilles de la hélice (10).

3. Dispositif selon la revendication 2, dans lequel une rainure longitudinale (13) est située dans le manchon coupant (1) et il y a un trou de traversée fileté (14), dont axis est perpendiculaire à l'axis d'un trou du boîtier (8), dans le boîtier (8), dans lequel il y a : une vis de réglage (15) et une vis de serrure (16) qui sont montées à travers et à l'opposé, dans le trou de traversée fileté (14).
